(19) Europäisches Patentamt
European Patent Office
Office européen des brevets

(11) **EP 3 025 763 B1**

(12) **FASCICULE DE BREVET EUROPEEN**

(45) Date de publication et mention de la délivrance du brevet:
**10.06.2020 Bulletin 2020/24**

(21) Numéro de dépôt: **15194565.6**

(22) Date de dépôt: **21.12.2007**

(51) Int Cl.:
*A61Q 19/08* (2006.01)   *A61Q 7/00* (2006.01)
*A61Q 17/04* (2006.01)   *A61Q 19/00* (2006.01)
*A61K 8/36* (2006.01)   *A61K 8/38* (2006.01)
*A61K 31/327* (2006.01)   *A61P 17/00* (2006.01)
*A61K 31/185* (2006.01)   *A61K 8/04* (2006.01)
*A61Q 5/00* (2006.01)   *A61K 8/67* (2006.01)
*A61K 8/73* (2006.01)   *A61K 31/203* (2006.01)
*A61K 8/11* (2006.01)   *A61K 8/44* (2006.01)
*A61K 8/81* (2006.01)   *A61K 9/06* (2006.01)
*A61K 47/44* (2017.01)

(54) **GEL CREME COMPRENANT AU MOINS UN RETINOIDE ET DU PEROXYDE DE BENZOYLE**

CREMEGEL, DAS MINDESTENS EIN RETINOID UND BENZOYLPEROXID UMFASST

CREAM GEL COMPRISING AT LEAST ONE RETINOID AND BENZOYL PEROXIDE

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **21.12.2006 FR 0655784**

(43) Date de publication de la demande:
**01.06.2016 Bulletin 2016/22**

(62) Numéro(s) de document de la (des) demande(s) initiale(s) en application de l'article 76 CBE:
**07872022.4 / 2 125 118**

(73) Titulaire: **Galderma Research & Development 06410 Biot (FR)**

(72) Inventeurs:
• **MALLARD, Claire 06250 MOUGINS (FR)**
• **LOUIS, Fabienne 06270 VILLENEUVE LOUBET (FR)**
• **AT, Emmanuelle 06600 ANTIBES (FR)**

(74) Mandataire: **Nederlandsch Octrooibureau P.O. Box 29720 2502 LS The Hague (NL)**

(56) Documents cités:
WO-A-81/00206   WO-A-93/20796
WO-A-03/055472   FR-A- 2 687 312
FR-A1- 2 225 167   US-A- 3 535 422
US-A- 4 189 501

• MARTIN B ET AL: "CHEMICAL STABILITY OF ADAPALENE AND TRETINOIN WHEN COMBINED WITH BENZOYL PEROXIDE IN PRESENCE AND IN ABSENCE OF VISBILE LIGHT AND ULTRAVIOLET RADIATION", BRITISH JOURNAL OF DERMATOLOGY, vol. 139, no. SUPPL 52, octobre 1998 (1998-10), pages 8-11, XP008007635, ISSN: 0007-0963

Remarques:
Le dossier contient des informations techniques présentées postérieurement au dépôt de la demande et ne figurant pas dans le présent fascicule.

EP 3 025 763 B1

**Description**

L'invention se rapporte à une composition sous forme de gel-crème comprenant, dans un milieu physiologiquement acceptable, de l'adapalène et du péroxyde de benzoyle dispersé.

L'utilisation de plusieurs classes de principes actifs est un outil thérapeutique auquel il est fréquemment fait recours, notamment pour le traitement de désordres dermatologiques. En effet, différents antifongiques comme les dérivés allylamines, les triazoles, les antibactériens ou antimicrobiens comme par exemple les antibiotiques, les quinolones et les imidazoles, sont classiquement associés dans le traitement de maladies dermatologiques. Il est également connu d'utiliser les péroxydes, les vitamines D et les rétinoïdes pour le traitement topique de diverses pathologies liées à la peau ou les muqueuses, en particulier l'acné.
La combinaison de plusieurs traitements locaux (antibiotiques, rétinoïdes, péroxydes, zinc) est également utilisée en dermatologie pour permettre d'augmenter l'efficacité des principes actifs et de diminuer leur toxicité (Cunliffe W.J., J. Dermatol. Treat., 2000, 11 (suppl2), S13-S14).
L'application multiple de différents produits dermatologiques peut être assez lourde et astreignante pour le patient.

On comprend donc l'intérêt de chercher à obtenir un nouveau traitement efficace sur les affections dermatologiques dans une composition stable offrant une bonne cosméticité, permettant une application unique et une utilisation agréable pour le patient.
Parmi cette panoplie de thérapeutiques proposée à l'homme du métier, rien ne l'encourageait à associer, dans la même composition, le péroxyde de benzoyle et un rétinoïde.

Toutefois, la formulation d'une telle composition pose plusieurs problèmes.

Tout d'abord, l'efficacité du péroxyde de benzoyle est liée à sa décomposition lorsqu'il est mis en contact avec la peau. En effet, ce sont les propriétés oxydantes des radicaux libres produits lors de cette décomposition qui conduisent à l'effet désiré. Aussi, afin de maintenir au péroxyde de benzoyle une efficacité optimale, il est important de prévenir sa décomposition avant utilisation, c'est à dire durant le stockage.

Or le péroxyde de benzoyle est un composé chimique instable qui rend difficile sa formulation dans des produits finis.

La solubilité et la stabilité du péroxyde de benzoyle ont été étudiées par Chellquist *et al.* dans l'éthanol, le propylène glycol et différents mélanges de polyéthylène glycol 400 (PEG 400) et d'eau (Chellquist E.M. et Gorman W.G., Pharm. Res., 1992, Vol 9: 1341-1346). Le péroxyde de benzoyle est particulièrement soluble dans le PEG400 et l'éthanol comme le montre le tableau suivant :

| Solvant | Solubilité du péroxyde de benzoyle (mg/g) |
|---|---|
| PEG 400 | 39.6 |
| Ethanol | 17.9 |
| Propylène Glycol | 2.95 |
| Propylène Glycol / Eau (75 :25) | 0.36 |
| Glycérine | 0.15 |
| Eau | 0.000155 |

Ce document précise par ailleurs que la stabilité du péroxyde de benzoyle est fortement influencée par la composition chimique de la formulation et par la température de stockage. Le péroxyde de benzoyle est extrêmement réactif et se dégrade en solution à basse température en raison de l'instabilité de sa liaison péroxyde.
Les auteurs constatent ainsi que le péroxyde de benzoyle en solution se dégrade plus ou moins rapidement dans tous les solvants étudiés en fonction du type de solvant et de sa concentration.
Les temps de dégradation du péroxyde de benzoyle dans le PEG 400 (0.5 mg/g), dans l'éthanol et dans le propylène glycol sont respectivement de 1,4 , 29 et 53 jours à 40°C. Une telle dégradation ne permet pas la préparation d'un produit destiné à la vente.

Il est connu par ailleurs que le péroxyde de benzoyle est plus stable dans l'eau et le propylène glycol lorsqu'il est en suspension (ie sous forme dispersée), puisqu'il n'est pas dégradé après 90 jours de conservation dans ces solvants. Ainsi, pour limiter le problème d'instabilité rapide du péroxyde de benzoyle en solution, il s'est avéré avantageux de formuler le péroxyde de benzoyle sous forme dispersée. Cependant, ce type de formulation n'est pas totalement satisfaisant dans la mesure où on constate toujours une dégradation du péroxyde de benzoyle dans le produit fini.

Une autre difficulté à surmonter pour la préparation d'une composition comprenant à la fois du péroxyde de benzoyle et un rétinoïde est que la plupart des rétinoïdes est particulièrement sensible à l'oxydation naturelle, à la lumière visible et aux ultra-violets, et le péroxyde de benzoyle étant un oxydant fort, la compatibilité chimique de ces composés dans une même formulation pose de nombreux problèmes de stabilité du point de vue physique et chimique.

Une étude de stabilité de deux rétinoïdes a été réalisée en combinant deux produits commercialisés, l'un contenant un rétinoïde (trétinoïne ou adapalène) et le second à base de péroxyde de benzoyle (B. Martin et al., Br.J.Dermatol. (1998) 139, (suppl.52), 8-11). La présence de la formulation à base de péroxyde de benzoyle provoque une dégradation très rapide des rétinoïdes sensibles à l'oxydation : on mesure que 50% de la trétinoïne se dégrade en 2 heures, et 95% en 24 heures. Dans la composition dans laquelle le rétinoïde est l'adapalène, aucune dégradation de de l'adapalène n'a été mesurée pendant 24 heures. Cette étude confirme que le péroxyde de benzoyle se dégrade et dégrade les rétinoïdes sensibles à l'oxydation au cours du temps en relarguant progressivement de l'acide benzoïque dans des produits finis.

Or il est clair que la dégradation du péroxyde de benzoyle et des rétinoïdes n'est pas souhaitable dans la mesure où elle nuit à l'efficacité de la composition les contenant.

La demande de brevet WO03/055472 décrit des compositions comprenant un rétinoïde, et notamment l'adapalène, du peroxyde de benzoyle dispersé ainsi qu'un agent gélifiant pH-indépendant, et qui se présente sous la forme d'un gel. Ces compositions sont des gels aqueux, et non pas des gels crèmes comprenant de 5 à 30 % en poids de phase grasse.

La publication B.Martin et al., Br.J.Dermatol. (1998), 139, (suppl.52), 8-11 précitée décrit le mélange extemporané d'un gel d'adapalène 0,1% (Differin 0,1%) avec une lotion de peroxyde de benzoyle à 10% (Cutacnyl 10% lotion). Ces compositions ne comprennent pas de phase grasse.

Rien n'incitait à associer ces deux agents actifs afin d'obtenir une composition stable de type gel-crème sachant qu'il était usuellement connu que la présence du péroxyde de benzoyle déstabilisait chimiquement et physiquement ce type de composition.

La formulation en gel crème du péroxyde de benzoyle et d'un rétinoïde peut être avantageuse pour les traitements topiques, tels que celui de l'acné, car tout en apportant l'émollience, elle évite notamment de laisser subsister un toucher trop gras sur la peau.

Or, une autre difficulté à surmonter pour la préparation d'une telle composition comprenant notamment des actifs dispersés tels que de l'adapalène et le péroxyde de benzoyle est la sédimentation des actifs. En effet, si le toucher « léger » d'une telle formulation est lié au fait que la phase externe soit aqueuse, il dépend aussi de sa composition et notamment de la présence d'épaississants. Or, dans les gels crèmes les épaississants de phase grasse tels que les cires, les alcools et esters gras solides sont fortement diminués au profit de gélifiants de phase aqueuse. Cependant, la plupart des gélifiants de phase aqueuse sont déstabilisés par l'acide benzoïque qui est libéré lors de la dégradation du péroxyde de benzoyle.

En effet, les agents épaississants les plus couramment utilisés pour la formulation de gels avec du péroxyde de benzoyle sont les polymères d'acide acrylique (Carbomer) et les celluloses seules ou associées à des silicates.

Or, l'utilisation de carbomères dans des compositions de type gel aqueux ne donne pas de bons résultats en terme de stabilité chimique du péroxyde de benzoyle et en terme de stabilité rhéologique. Comme décrit par Bollinger (Bollinger, Journal of Pharmaceutical Science, 1977, vol 5), il a été observé une perte de 5 à 20% de péroxyde de benzoyle au bout de 2 mois à 40°C selon le neutralisant du carbomère utilisé. De plus, la libération d'acide benzoïque provoque la dépolymérisation des carbomères, donnant une chute de viscosité pouvant entraîner un déphasage.

Dans d'autres gels constitués d'un mélange d'hydroxypropylcellulose et de silicate d'aluminium et de magnésium, on observe également une chute de viscosité au cours du temps qui entraîne une sédimentation des actifs en suspension et une hétérogénéité de la dispersion dans le produit fini.

Cette instabilité des gels de péroxyde de benzoyle nuit à leur efficacité et à leur cosméticité et il est fortement probable qu'on la retrouve dans les gels crèmes. Un produit fini, en particulier lorsqu'il s'agit de compositions pharmaceutiques ou cosmétiques, doit conserver tout au long de sa durée de vie des critères physico-chimiques précis permettant de garantir sa qualité pharmaceutique ou cosmétique, respectivement. Parmi ces critères, il est nécessaire que les propriétés rhéologiques soient conservées. Elles définissent le comportement et la texture de la composition lors de l'application, mais aussi les propriétés de libération du principe actif [Rapport commission SFSTP 1998] et l'homogénéité du produit lorsque les principes actifs y sont présents à l'état dispersé.

Il existe donc le besoin de disposer d'un gel crème physiquement et chimiquement stable comprenant du péroxyde de benzoyle et un rétinoïde.

Or, la Demanderesse a réalisé une composition satisfaisant ce besoin. Une telle composition est un gel crème qui comprend notamment-:

- du péroxyde de benzoyle dispersé, notamment sous forme libre ou encapsulée,
- de l'adapalène,
- au moins un composé lipophile composant la phase grasse,
- et au moins un gélifiant pH-indépendant ayant une bonne stabilité physique, c'est-à-dire ne présentant pas de chute de viscosité dans le temps et à des températures comprises entre 4 et 40°C, et maintenant une bonne stabilité chimique des deux actifs (péroxyde de benzoyle et adapalène), c'est-à-dire qu'on ne constate pas de dégradation des actifs dans le temps et à des températures comprises entre 4 et 40°.

Les compositions de la présente invention peuvent se présenter sous toutes les formes galéniques normalement utilisés pour une application topique et notamment sous forme de gel crème de consistance semi-liquide du type lait à consistance solide du type crème, obtenus par dispersion d'une phase grasse dans une phase aqueuse (H/E). L'homme du métier veillera à choisir les excipients constituant les compositions selon l'invention en fonction de la consistance souhaitée et de manière à ce que les propriétés avantageuses de la composition selon l'invention soient respectées.

La composition selon l'invention peut notamment comprendre, outre de l'adaplène, du péroxyde de benzoyle, une phase grasse et au moins un gélifiant pH-indépendant un ou plusieurs des ingrédients suivants :

a) un ou plusieurs agents mouillants,
b) un ou plusieurs agents chélatants,
c) une phase aqueuse,
d) un ou plusieurs additifs.

La Demanderesse a également découvert de manière surprenante que l'on pouvait obtenir une parfaite dispersion des principes actifs en suivant un procédé de préparation particulier. Ce procédé de préparation permet d'obtenir une granulométrie optimale et une dispersion homogène des deux actifs dans la composition, tout en garantissant la stabilité physique du produit.

L'invention se rapporte donc à une composition sous la forme d'un gel-crème comprenant, dans un milieu physiologiquement acceptable, de l'adapalène et du péroxyde de benzoyle dispersé, telle que définie dans la revendication 1.

La composition selon l'invention est sous la forme d'un gel crème aqueux. Le gel-crème se caractérise par la présence de gélifiants de phase aqueuse et d'une phase grasse. Par contre, il n'y a pas d'émulsionnant(s) ce qui différencie les gel-crèmes des émulsions.

Par émulsionnants on entend des composés amphiphiles qui possèdent une partie hydrophobe ayant une affinité pour l'huile et une partie hydrophile ayant une affinité pour l'eau créant ainsi un lien entre les deux phases. Les émulsionnants ioniques ou non ioniques stabilisent donc les émulsions H/E en s'adsorbant à l'interface et en formant des couches lamellaires de cristaux liquides.

Particulièrement, la composition selon l'invention est stable physiquement et chimiquement.

Par milieu physiologiquement acceptable, on entend un milieu compatible avec une application topique sur la peau, les phanères et/ou les muqueuses.

La composition selon l'invention contient un rétinoïde. choisi parmi l'adapalène ainsi que ses sels. On entend par sels de l'adapalène, les sels formés avec une base pharmaceutiquement acceptable, notamment des bases minérales telles que la soude, la potasse et l'ammoniaque ou des bases organiques telles que la lysine, l'arginine, la N-méthyl-glucamine. On entend également par sels de de l'adapalène les sels formés avec des amines grasses telles que la dioctylamine et la stéarylamine.

La composition selon l'invention comprend entre 0.001 et 5% et avantageusement entre 0.01 et 1 % en poids d'adapalène par rapport au poids total de la composition, préférentiellement entre 0.01 et 0.5%, de préférence, entre 0.1 et 0.4 % en poids d'adapalène, encore plus préférentiellement 0.3 % en poids d'adapalène.

Le péroxyde de benzoyle pourra aussi bien être utilisé sous la forme libre ou bien sous une forme encapsulée par exemple sous forme adsorbée sur, ou absorbée dans tout support poreux. Il peut s'agir par exemple de péroxyde de benzoyle encapsulé dans un système polymérique constitué de microsphères poreuses, comme par exemple des microéponges vendues sous le nom de Microsponges P009A Benzoyle péroxyde par la société Amcol.

Par exemple, dans les compositions pour le traitement de l'acné, le péroxyde de benzoyle est utilisé, de préférence, à des concentrations allant de 2 à 10 % en poids et plus particulièrement de 2,5 à 5 % en poids par rapport au poids total de la composition. L'adapalène est quant à lui utilisé dans ce type de composition à des concentrations allant généralement de 0,01 à 1 % en poids par rapport au poids total de la composition.

De façon avantageuse, la granulométrie de l'adapalène et du péroxyde de benzoyle est telle qu'au moins 80% en nombre des particules, et de préférence au moins 90% en nombre des particules, ont un diamètre inférieur à 25 $\mu$m et au moins 99% en nombre des particules ont un diamètre inférieur à 100 $\mu$m.

Le gel crème selon l'invention comprend un ou plusieurs agents gélifiants pH indépendants. Par gélifiant pH indépendant, on entend un gélifiant capable de conférer une viscosité suffisante à la composition pour maintenir en suspension l'adapalène et le péroxyde de benzoyle, même sous l'influence d'une variation de pH due au relarguage d'acide benzoïque par le péroxyde de benzoyle.

A titre d'exemples non limitatifs d'agents gélifiants pH-indépendants pouvant entrer dans les compositions selon l'invention, on peut citer l'Acrylates/C10-30 Alkyl Acrylate Crosspolymer vendu sous le nom de Pemulen TR-1 ou Pemulen TR-2 par la société Noveon , les carbomers dits non sensibles aux électrolytes, vendus sous le nom, d'Ultrez 20®, Carbopol 1382 ou de Carbopol ETD2020NF® vendu par la société Noveon, les polysaccharides avec à titre d'exemples non limitatifs la gomme de xanthane telle que le Xantural180® vendu par la société Kelco, la gomme guar, les chitosans, les carraghénanes en particulier réparties sous quatre grandes familles : κ, λ, β, ω tels que les Viscarin® et les Gelcarin® commercialisés par la société IMCD, la cellulose et ses dérivés tels que l'hydroxypropylméthylcellulose en particulier le produit vendu sous le nom de Methocel E4 premium par la société Dow Chemical ou l'hydroxyéthylcellulose, en particulier, le produit vendu sous le nom de Natrosol HHX 250® par la société Aqualon ou encore le produit « microcrystalline cellulose and carboxymethyl cellulose sodium » vendu sous le nom d'Avicel CL-611 par la société FMC Biopolymer, la famille des silicates d'aluminium et de magnésium tels que le Veegum K vendu par la société Vanderbilt ,la famille des polymères acryliques couplés à des chaînes hydrophobes tel que le PEG-150/decyl/SMDI copolymer vendu sous le nom de Aculyn 44 (polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%)), la famille des amidons modifiés tels que l'amidon de pomme de terre modifié vendu sous le nom de Structure Solanace ou bien leurs mélanges et les gélifiants de la famille des polyacrylamides tels que le mélange Sodium acryloyldimethyltaurate copolymer / isohexadecane / polysorbate 80 vendu sous le nom Simulgel 600PHA par la société Seppic, le mélange polyacrylamide / isoparaffine C13-14 / laureth-7 comme, par exemple, celui vendu sous le nom de Sepigel 305 par la société Seppic.

Les agents gélifiants préférés sont issus de la famille des polyacrylamides tel que le Simulgel 600PHA ou le Sepigel 305 ; des carbomers dits non sensibles aux électrolytes tel que le Carbopol ETD2020 NF; des polysaccharides tel que la gomme de xanthane ; des dérivés cellulose tel que l'hydroxypropylméthylcellulose ou l'hydroxyéthylcellulose ; des aluminium magnésium silicates seuls ou en mélange.

Le gélifiant tel que décrit ci-dessus peut être utilisé aux concentrations préférentielles allant de 0.01 à 15 % et, plus préférentiellement, allant de 0,1 à 5 %.

Parmi les agents chélatants, on peut citer à titre d'exemples non limitatifs l'acide éthylène diamine tétraacétique (EDTA), l'acide diéthylène triamine pentaacétique (DTPA), l'acide éthylène diamine-di (O-hydroxyphényl acétique) (EDDHA), l'acide hydroxy-2-éthylène diamine triacétique (HEDTA), l'acide éthyldiamine-di (O-hydroxy-p-méthyl phényl) acétique (EDDHMA) et l'acide éthylène diamine-di (5-carboxy-2-hydroxyphényl) acétique (EDDCHA).

A titre d'agent chélatant préféré, on peut citer l'acide éthylène diamine tétraacétique (EDTA).

Les concentrations d'agent chélatant peuvent varier de 0% à 1,5% préférentiellement 0,05% à 0.5% en poids par rapport au poids total de la composition.

Les compositions de l'invention peuvent comprendre un ou plusieurs agents mouillants à des concentrations de 0 à 10% en poids par rapport au poids total de la composition. Lorsque ces ingrédients sont présents dans la composition, ils sont à des concentrations allant de 0,001 à 10%, de manière préférée 0,1% à 7% d'agent(s) mouillant(s) et plus préférentiellement encore allant de 2 à 7 % en poids par rapport au poids total de la composition. Ils doivent être non solubilisants des actifs au pourcentage utilisé, ne pas provoquer de réactions exothermiques néfastes pour le péroxyde de benzoyle, aider à la bonne dispersion des actifs et avoir des propriétés anti-mousses. Le pouvoir mouillant est la tendance d'un liquide à s'étaler sur une surface.

De préférence, il s'agit d'agents mouillants pouvant présenter une HLB (Hydrophilic Lipophilic Balance) de 7 à 16. A titre d'exemple non limitatif, on citera les Poloxamers et plus particulièrement le Synperonic PE/L44 et/ou le Synperonic PE/L62 vendus par la société Uniqema, des glycols tels que le propylene glycol, le dipropylène glycol, le propylène glycol dipélargonate, le lauroglycol, l'éthoxydiglycol, des esters de sorbitan tels que le POE(20) sorbitan monooléate, vendu sous le nom de Tween 80 par la société Uniqema et le POE(20) sorbitan monostéarate vendu sous le nom de « Tween 60 » par la société Uniqema, les éthers d'alcools gras tel que le Ceteareth-20 vendu sous le nom d' Eumulgin B2 par la société Cognis, les esters de glycérol tel que le monostéarate de glycérol vendu sous le nom de « Cutina GMS » par la société Cognis, le Polyoxyethylene (21) stearyl éther vendu sous le nom de Brij 721 par la société Uniqema, le methyl glucose sesquistearate vendu sous le nom de Glucate SS par la société Noveon, le PEG-20 Methyl glucose sesquistearate vendu sous le nom de Glucamate SSE-20 par la société Noveon.

Parmi les agents mouillants, on utilise préférentiellement, des agents mouillants pouvant présenter préférentiellement une HLB de 10 à14, sans que cette liste soit limitative, des composés de la famille des Poloxamers et plus particulièrement le Synperonic PE/L44 et/ou le Synperonic PE/L62 ou des glycols tels que le propylène glycol, le dipropylène glycol, le propylène glycol dipélargonate, le lauroglycol, l'éthoxydiglycol.

Les agents mouillants particulièrement préférés sont le propylène glycol ou le Synperonic PE/L44 (Polyethylene-polypropylene glycol; Polyoxyethylene-Polyoxypropylene Block Copolymer).

Selon l'invention, d'une manière avantageuse, le gel-crème peut également comprendre un ou plusieurs agents mouillants.

La composition selon l'invention comprend également une phase grasse. Cette phase grasse comprend des composés lipophiles seuls ou en mélange choisis parmi les huiles végétales, minérales, animales ou synthétiques, des huiles de silicones, et leurs mélanges.

Comme exemple d'huile minérale, on peut citer par exemple des huiles de paraffine de différentes viscosités telles que le Primol 352®, le Marcol 82®, Marcol 152® vendus par la société Esso.

Comme huile végétale, on peut citer l'huile d'amande douce, l'huile de palme, l'huile de soja, l'huile de sésame, l'huile de tournesol.

Comme huile animale, on peut citer la lanoline, le squalène, l'huile de poisson, l'huile de vison avec comme dérivé le squalane vendu sous le nom Cosbiol® par la société Laserson.

Comme huile synthétique, on peut citer un ester tel que le cétéaryl isononanoate comme le produit vendu sous le nom de Cetiol SN PH® par la société Cognis France, le palmitate d'isopropyle comme le produit vendu sous le nom de Crodamol IPP® par la société Croda, le diisopropyl adipate vendu sous le nom de Crodamol DA par la société Croda, le caprylique/caprique triglycéride tel que Miglyol 812® vendu par la société Hüls / Univar.

Comme huile de silicone volatil ou non volatil, on peut citer des diméthicones comme les produits vendus sous le nom de Q7-9120 silicone fluid de viscosité comprise entre 20cst et 12500 cst ou le produit vendu sous le nom le ST-Cyclomethicone 5NF® par la société Dow Corning.

On pourra également mettre des corps gras solides tel que des cires naturelles ou synthétiques. Dans ce cas, l'homme du métier adaptera la température de chauffage de la préparation en fonction de la présence ou non de ces solides.

Pour la composition selon l'invention, les huiles synthétiques et les huiles de silicone et plus particulièrement le Marcol 152® et le ST-Cyclomethicone 5 NF® sont préférées.

La phase aqueuse du gel crème selon l'invention comprend de l'eau. Cette eau peut notamment être une eau florale telle que l'eau de bleuet, ou une eau thermale ou minérale naturelle, par exemple choisie parmi l'eau de Vittel, les eaux du bassin de Vichy, l'eau d'Uriage, l'eau de la Roche Posay, l'eau d'Avène ou l'eau d'Aix les Bains.

Ladite phase aqueuse peut être présente à une teneur comprise entre 10 et 90 % en poids par rapport au poids total de la composition, de préférence comprise entre 20 et 80 % en poids.

La composition peut comprendre en outre tout additif usuellement utilisé dans le domaine cosmétique ou pharmaceutique, tel qu'un stabilisant du benzoyle peroxide (à titre d'exemple le docusate de sodium, le sodium C14-16 oléfine sulfonate), des agents neutralisants type bases ou acides usuels minéraux ou organiques (à titre d'exemple, la triethanolamine, la solution de soude 10%, le tampon acide citrique/sodium citrate, le tampon acide succinique/succinate de sodium), , des antioxydants, des filtres solaires, des conservateurs, des charges, des électrolytes, des humectants et/ou emollients, des colorants , des parfums, des huiles essentielles, des actifs cosmétiques, des hydratants, des vitamines, des acides gras essentiels, des sphingolipides, des composés auto-bronzants tels que la DHA, des agents apaisants et protecteurs de la peau tels que l'allantoïne. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées.

Ces additifs peuvent être présents dans la composition à raison de 0,001 à 20 % en poids par rapport au poids total de la composition.

On peut citer à titre d'exemples de conservateurs le chlorure de benzalkonium, le bronopol, la chlorhexidine, le chlorocrésol et ses dérivés, l'alcool éthylique, l'alcool phénéthylique, le phénoxyéthanol, le sorbate de potassium, la diazolidinylurée, le chlorure de benzalkonium, le phénoxyéthanol, l'alcool benzylique, la diazolidinylurée, les parabens, ou leurs mélanges.

On peut citer comme exemples d'agents humectants et/ou émollients, la glycérine, le sorbitol, les sucres (à titre d'exemple le glucose, le lactose), les PEG (à titre d'exemple le Lutrol E400), l'urée, les acides aminés (à titre d'exemple la sérine, la citrulline, l'alanine).

En particulier, l'invention concerne une composition pharmaceutique ou cosmétique sous forme de gel-crème comprenant, dans un milieu physiologiquement acceptable et compatible avec l'application topique sur la peau, les phanères ou les muqueuses les ingrédients (exprimé en pourcentage en poids) suivants:

- de 0,001 à 5%, préférentiellement de 0,01% à 0,5% d'adapalène;
- 0,025 à 10 %, préférentiellement 2 à 10 %, de péroxyde de benzoyle ;
- de 30% à 95% préférentiellement 50% à 85% d'eau;
- de 0,01% à 15% préférentiellement 0,1% à 5% d'un ou plusieurs agents gélifiants pH-indépendants ;
- de 5% à 30% de phase grasse ;
- de 0% à 1,5% préférentiellement 0,05% à 0.5% d'agents chélatants ;
- de 0% à 10% préférentiellement 2% à 7 % d'un ou plusieurs agents mouillants ;
- de 0% à 3% préférentiellement 0,05% à 1% d'agents conservateurs ;
- de 0% à 20% préférentiellement 2% à 15% d'agents humectants et/ou émollients ;
- de 0% à 3% préférentiellement 0,05% à 2% d'agents stabilisants ;
- de 0% à 10% préférentiellement 0,1% à 5% d'agents neutralisants.

La présente invention a aussi pour objet la composition telle que décrite précédemment à titre de médicament.

L'invention se rapporte également à l'utilisation de la nouvelle composition telle que décrite précédemment en cosmétique et en dermatologie.

De part l'activité kératolytique, bactéricide et anti-inflammatoire du péroxyde de benzoyle et l'activité marquée des rétinoïdes dans les domaines de la différentiation et de la prolifération cellulaire, les compositions de l'invention conviennent particulièrement bien dans les domaines thérapeutiques suivants :

1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle, l'hidradenite supurative,

2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),

3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation telles que les folliculites,

4) pour traiter toutes les proliférations dermiques ou épidermiques qu'elles soient bénignes ou malignes, qu'elles soient ou non d'origine virale telles que verrues vulgaires, les verrues planes, le molluscum contagiosum, et l'épidermodysplasie verruciforme, les papillomatoses orales ou florides et les proliférations pouvant être induites par les ultra-violets notamment dans le cas des kératoses actiniques,

5) pour réparer ou lutter contre le vieillissement de la peau, qu'il soit photo-induit ou chronologique, ou pour réduire les pigmentations, ou toutes pathologies associées au vieillissement chronologique ou actinique,

6) pour traiter de manière préventive ou curative les troubles de la cicatrisation, les ulcères cutanés, pour prévenir ou pour réparer les vergetures, ou encore pour favoriser la cicatrisation,

7) pour lutter contre les troubles de la fonction sébacée tels que l'hyperséborrhée de l'acné ou la séborrhée simple,

8) dans le traitement de toute affection d'origine fongique au niveau cutané tel que le tinea pedis et le tinea versicolor,

9) dans le traitement d'affections dermatologiques à composante immunologique,

10) dans le traitement de désordres cutanés dus à une exposition aux rayonnements U.V., et

11) dans le traitement d'affections dermatologiques liées à une inflammation ou une infection des tissus environnants le follicule pileux, notamment dues à une colonisation ou infection microbienne notamment l'impétigo, la dermite seborrhéique, la folliculite, le sycosis barbae ou impliquant tout autre agent bactérien ou fongique.

Les compositions selon l'invention sont particulièrement adaptées au traitement, de manière préventive ou curative, des acnés vulgaires.

Un objet de l'invention se rapporte également à la préparation d'une composition pharmaceutique destinée à la prévention et/ou au traitement des affections dermatologiques liées à des désordres de la différentiation et/ou de la prolifération cellulaire et/ou de la kératinisation, de préférence les acnés vulgaires.

Les compositions selon l'invention trouvent aussi une application dans l'hygiène corporelle et capillaire.

La présente invention se rapporte ainsi également à l'utilisation cosmétique d'une composition selon l'invention pour le traitement des peaux à tendance acnéique, pour faire repousser les cheveux ou éviter leur chute, pour lutter contre l'aspect gras de la peau ou des cheveux, dans la protection contre les aspects néfastes du soleil, ou pour prévenir et/ou lutter contre le vieillissement photo-induit ou chronologique.

Préférentiellement, lesdites compositions selon l'invention sont administrées par voie topique.

L'invention a également pour objet un procédé de préparation d'une composition telle que décrite précédemment. Un tel procédé est caractérisé en ce qu'il comprend une étape de mélange d'un milieu physiologiquement acceptable avec de l'adapalène et au moins du péroxyde de benzoyle.

L'introduction des autres excipients et additifs éventuels se fera en fonction de la nature chimique des composés et de la forme galénique choisie.

D'une manière générale, la préparation d'une composition selon l'invention se fait ainsi selon le procédé principal suivant:

a) mélange de l'adapalène avec de l'eau jusqu'à parfaite dispersion, afin d'obtenir la phase active 1;

b) mélange du péroxyde de benzoyle avec de l'eau jusqu'à parfaite dispersion, afin d'obtenir la phase active 2;

c) mélange d'au moins un agent gélifiant pH-indépendant avec de l'eau, optionnellement un ou plusieurs agents chélatants, un ou plusieurs conservateurs, un ou plusieurs humectants et/ou émollients, un ou plusieurs agents stabilisants et les additifs hydrophiles afin d'obtenir la phase aqueuse ;

d) optionnellement, mélange d'au moins deux composés lipophiles afin d'obtenir la phase grasse ;

e) mélange des 2 phases actives obtenues en a) et b) pour obtenir une phase active unique ;

f) introduction de la phase active unique obtenue en e) dans la phase aqueuse obtenue en c) ;

g) introduction du composé unique de la phase grasse ou optionnellement de la phase grasse obtenue en d) afin d'obtenir un gel-crème ;

h) si nécessaire, les additifs thermosensibles sont ajoutés ;

i) si nécessaire, un agent de neutralisation du gélifiant est introduit dans le gel-crème obtenu en h ;

j) si nécessaire un complément d'eau est ajouté.

D'une manière générale, la préparation d'une composition selon l'invention se fait ainsi selon le procédé alternatif suivant:
a') Les étapes a) et b) sont regroupées de manière à obtenir l'étape a') qui correspond au mélange de l'adapalène, du peroxyde de benzoyle avec de l'eau et au moins un agent mouillant, jusqu'à parfaite dispersion, afin d'obtenir la phase active unique;
Les étapes c), d), f), g), h), i), j), du procédé principal restent inchangées. L'étape e) est quant à elle supprimée.
Selon un mode particulier la préparation d'une composition selon l'invention se fait à titre d'exemple selon le procédé principal suivant:

a) On mélange l'adapalène avec au moins un agent mouillant, dans de l'eau, jusqu'à ce que l'adapalène soit parfaitement dispersé, afin d'obtenir la phase active 1;

b) On mélange le péroxyde de benzoyle avec au moins un agent mouillant, dans de l'eau, jusqu'à ce qu'il soit parfaitement dispersé, afin d'obtenir la phase active 2;

c) On solubilise dans l'eau, sous agitation, si nécessaire à chaud, un ou plusieurs agents gélifiants pH-indépendants (à l'exception du polyacrylamide) et optionnellement, un ou plusieurs agents chélatants, un ou plusieurs conservateurs, un ou plusieurs humectants et/ou émollients, un ou plusieurs agents stabilisants et les additifs hydrophiles non thermosensibles. On maintient l'agitation et l'éventuel chauffage jusqu'à homogénéité pour obtenir la phase aqueuse ;

d) Optionnellement, on mélange si nécessaire à chaud au moins des huiles, et éventuellement des corps gras solides, et des conservateurs et les additifs lipophiles non thermosensibles jusqu'à homogénéité afin d'obtenir la phase grasse ;

e) On mélange les phases actives 1 et 2 de manière à obtenir une phase active unique ;

f) On additionne la phase active unique obtenue en e) à la phase aqueuse obtenue en c) ;

g) Optionnellement, on introduit le polyacrylamide à la phase obtenue en f) ;

h) L'unique constituant de phase grasse ou optionnellement, ladite phase grasse obtenue en d) est introduit dans la phase obtenue en f) ou g) afin d'obtenir un gel crème;

i) Si nécessaire, les additifs thermosensibles sont ajoutés ;

j) Si nécessaire, un agent de neutralisation du gélifiant est introduit dans le gel crème obtenu en f) afin d'obtenir le pH désiré,

k) Si nécessaire un complément d'eau est ajouté.

En particulier, la préparation d'une composition selon l'invention se fait ainsi selon le procédé alternatif suivant:
a') Les étapes a) et b) sont regroupées de manière à obtenir l'étape a') qui correspond au mélange de l'adapalène, du peroxyde de benzoyle avec de l'eau et au moins un agent mouillant, jusqu'à parfaite dispersion, afin d'obtenir la phase active unique;
Les étapes c), d), f), g), h), i), j), k) du procédé principal restent inchangées. L'étape e) est quant à elle supprimée.
Plus précisément, à titre d'exemple le procédé principal de préparation de la composition selon l'invention comprend les étapes suivantes:

**Etape a:** Préparation de la phase active 1:
Dans un bêcher, on pèse, le principe actif (adapalène), une partie de l'eau purifiée, le ou les agents mouillants (type propylène glycol, Synperonic PE/L62, Synperonic PE/L44). On les disperse sous agitation jusqu'à parfaite dispersion.

**Etape b:** Préparation de la phase active 2:
Dans un bêcher, on pèse, le principe actif (péroxyde de benzoyle), une partie de l'eau purifiée, les ou les agents mouillants (type propylène glycol, Synperonic PE/L62, Synperonic PE/L44). On les disperse sous agitation jusqu'à parfaite dispersion.

**Etape c:** Préparation de la phase aqueuse:
Dans le bêcher, on introduit sous agitation, si nécessaire à chaud, le reste d'eau purifiée, le ou les gélifiants pH-indépendants (à l'exception du Simulgel 600PHA) et optionnellement un ou plusieurs agents chélatants (type EDTA), un ou plusieurs humectants et/ou émollients (type glycérine), un ou plusieurs agents stabilisants (type docusate de sodium), un ou plusieurs conservateurs (type méthyl parabène), les additifs hydrophiles non thermosensibles. L'agitation et l'éventuel chauffage sont maintenus jusqu'à parfaite homogénéité ;

**Etape d :** Préparation de la phase grasse (optionnelle):
Dans un bêcher, les composés huileux (type olépal isostéarique, Cetiol SN, Crodamol DA, Speziol C18, Miglyol 812, Cosbiol) les éventuels additifs lipophiles non thermosensibles si chauffage et éventuellement les conservateurs (type phénoxyéthanol, propyl parabène). Le mélange est chauffé jusqu'à homogénéisation et on introduit le silicone volatile si ce dernier est présent dans la composition ;

**Etape e :** Mélange des phases actives
A une température inférieure à 40°C, les deux phases actives obtenues respectivement en a) et b) sont mélangées, l'agitation est maintenue jusqu'à parfaite homogénéisation ;

**Etape f :** Introduction de la phase active unique dans la phase aqueuse
La phase active unique obtenue à l'étape e) est introduite dans la phase aqueuse obtenue à l'étape c) ;

**Etape g** (optionnelle): Addition du Simulgel 600PHA
On introduit sous agitation le Simulgel 600PHA à la phase obtenue en f). Maintenir l'agitation jusqu'à parfaite dispersion ;

**Etape h :** Addition de l'huile ou de la phase grasse obtenue en d)
L'unique constituant de phase grasse ou optionnellement la phase grasse obtenue à l'étape d) est introduit dans le mélange obtenu en f) ou g) ;

**Etape i** (optionnelle): Ajout des additifs thermosensibles
A une température inférieure à 40°C, on introduit sous agitation les éventuels additifs. Maintenir l'agitation jusqu'à parfaite homogénéité

**Etape j** (optionnelle): Neutralisation :
A une température inférieure à 40°C, l'agent de neutralisation du gélifiant (type triéthanoloamine, la solution de soude 10%, le tampon acide citrique/sodium citrate, le tampon acide succinique/succinate de sodium) ou le tampon pH est introduit si nécessaire, jusqu'au pH désiré. Le produit prend alors une consistance plus épaisse. Si nécessaire, l'ajustement à 100% en eau est effectué. Le produit est homogénéisé une dernière fois afin de s'assurer de la bonne dispersion des principes actifs Adapalène et péroxyde de benzoyle (observation microscopique révélant une dispersion homogène et sans agrégats), puis le produit est conditionné.

**Etape k :** Correction de la perte en eau :

On calcule la perte en eau lors de la réalisation du produit et on rajoute sous agitation l'eau perdue, on maintien l'agitation jusqu'à parfaite homogénéité.

Plus précisément, à titre d'exemple, le procédé alternatif de préparation de la composition selon l'invention comprend les étapes suivantes:

**Etape a':** Préparation de la phase active unique:

Les étapes a) et b) du procédé principal sont regroupées de manière à obtenir l'étape a') qui correspond au mélange de l'adapalène, du peroxyde de benzoyle avec de l'eau et au moins un agent mouillant, jusqu'à parfaite dispersion, afin d'obtenir la phase active unique;

Les étapes c), d), f), g), h), i), j), k) du procédé principal restent inchangées. L'étape e) est quant à elle supprimée.

La présente invention va maintenant être illustrée au moyen des exemples et des données de stabilité physique et chimique suivants.

Les exemples de formulations ci-dessous permettent d'illustrer les compositions selon l'invention.

On entend par stabilité physique des formulations, la réalisation d'une observation macroscopique et microscopique à température ambiante, 40°C réalisée à T1 mois, T2 mois.

L'observation microscopique permet d'évaluer la qualité de la dispersion des deux actifs. L'Adapalène est observé en lumière fluorescente alors que le benzoyl peroxide est observé en lumière polarisée.

La caractérisation du produit fini est complétée par une mesure du seuil d'écoulement et de viscosité.

Pour la mesure du seuil d'écoulement, un rhéomètre HAAKE de type VT550 avec un mobile de mesure SVDIN est utilisé.

Les rhéogrammes sont réalisés à 25°C, à la vitesse de cisaillement de 4 $s^{-1}$, 20$s^{-1}$ et 100$s^{-1}$ ($\gamma$) et en mesurant la contrainte de cisaillement. Par seuil d'écoulement ($\tau 0$ exprimé en Pascal) on entend la force nécessaire (contrainte de cisaillement minimum) pour vaincre les forces de cohésion de type Van der Waals et provoquer l'écoulement. Le seuil d'écoulement est assimilé à la valeur trouvée à la vitesse de cisaillement de 4s-1.

Pour la mesure de la viscosité, les viscosimètres Brookfield RVDVII+ ou LVDVII+ sont utilisés. Les gammes de viscosités mesurables avec les 2 types de Brookfield sont les suivantes :

$$RVDVII+ : 100cP - 40McP$$

$$LVDVII+ : 15cP - 6McP$$

La stabilité chimique est assurée par un le dosage HPLC des actifs.

Le résultat est exprimé en g/g d'adapalène et de benzoyl peroxide et en % par rapport au titre attendu.

**Exemple 1 : Formulation de type gel crème contenant de l'adapalène à 0.1% et du péroxyde de benzoyle à 2.5%**

La formule est préparée selon le mode opératoire décrit ci-dessus :

| Constituants | Teneur (% m/m) |
|---|---|
| Péroxyde de benzoyle | 2,50 |
| Adapalène | 0.10 |
| Propylène glycol | 5,00 |
| Synperonic PE/L44 | 0,20 |
| EDTA | 0,10 |
| Glycérine | 5,00 |
| Xantural 180 | 0,10 |
| Carbopol Ultrez 20 | 0.70 |
| Marcol 152 | 7,00 |
| Eau purifiée | qsp 100% |
| Sodium Hydroxyde 10% m/m | qsp pH 5,5±0,5 |

Données de stabilités :

➤ Stabilité physique :

| Caractérisations à T0 | |
|---|---|
| Aspect macroscopique | Gel crème blanc |
| Aspect microscopique | Dispersion des actifs sans agrégats supérieurs à 100$\mu$m. |
| pH | 5.144 |
| Données de viscosité | Haake (4s-1/20s-1/100s-1) | 94/123/187 |
| | Brookfield RVDVII+ (S28 ; 5rpm) | 65620cP |

➤ Stabilité Chimique :

| | | T+1mois | T+2mois | T+3mois |
|---|---|---|---|---|
| Aspect macroscopique | TA | Identique à T0 | Identique à T0 | Identique à T0 |
| | 40°C | Identique à T0 | Identique à T0 | Identique à T0 |
| Aspect microscopique | TA | Identique à T0 | Identique à T0 | Identique à T0 |
| | 40°C | Identique à T0 | Identique à T0 | Identique à T0 |
| pH | TA | 5.10 | 5.03 | 5.09 |
| | 40°C | 4.96 | 4.74 | 4.59 |
| Rhéologie Haake 4s-1/20s-1/100s-1 | | 89/121/172 | 87/117/1 68 | NR |
| Viscosité Brookfield RVDVII+ (S 28 ; 5rpm) | | 65775cP | 63820 cP | 67505Cp |

➭ **Adapalène**

| Temps→ Conditions de stabilité↓ | | T0 | T+1mois | T+2mois |
|---|---|---|---|---|
| TA | g/g | 0.10 | 0.10 | 0.10 |
| | % titre attendu | 100 | 100 | 100 |
| 40°C | g/g | NA | 0.10 | 0.11 |
| | % titre attendu | NA | 100 | 110 |

➭ **Benzoyl peroxide**
| Temps→ Conditions stabilité↓ | | T0 | T+1mois | T+2mois |
|---|---|---|---|---|
| TA | g/g | 2.7 | 2.7 | 2.7 |
| | % titre attendu | 108 | 108 | 108 |

(suite)

| Temps→ | | T0 | T+1mois | T+2mois |
|---|---|---|---|---|
| Conditions stabilité↓ | | | | |
| 40°C | g/g | NA | 2.6 | 2.6 |
| | % titre attendu | NA | 104 | 104 |

## Exemple 2 : Formulation de type gel crème épais contenant de l'adapalène à 0.1% et du péroxyde de benzoyle à 2.5%

La formule est préparée selon le mode opératoire décrit ci-dessus :

➢ Stabilité physique :

| Constituants | Teneur (% m/m) |
|---|---|
| Péroxyde de benzoyle | 2.50 |
| Adapalène | 0.10 |
| Propylène glycol | 6,00 |
| Synperonic PE/L44 | 0,20 |
| Glycérine | 5,00 |
| ST-Cyclomethicone 5NF | 7,00 |
| Simulgel 600 PHA | 4,00 |
| Eau purifiée | qsp 100% |

| Caractérisations à T0 | | |
|---|---|---|
| Aspect macroscopique | | Gel crème blanc |
| Aspect microscopique | | Dispersion des actifs sans agrégats supérieurs à 100 $\mu$m. |
| pH | | 3.542 |
| Données de viscosité | Haake (4s-1/20s-1/100s-1) | 236/296/449 |
| | Brookfield RVDVII+ (S29 ; 5rpm) | 164650cP |

➢ Stabilité Chimique :

| | | T+1mois | T+2mois | T+3mois |
|---|---|---|---|---|
| Aspect macroscopique | TA | Identique à T0 | Identique à T0 | Identique à T0 |
| | 40°C | Identique à T0 | Identique à T0 | Identique à T0 |
| Aspect microscopique | TA | Identique à T0 | Identique à T0 | Identique à T0 |
| | 40°C | Identique à T0 | Identique à T0 | Identique à T0 |
| pH | TA | 3.47 | 3.36 | 3.50 |
| | 40°C | 3.31 | 3.17 | 3.27 |
| Rhéologie Haake 4s-1/ 20s-1/100s-1 | | 223/286/ 389 | 201/268/ 334 | NR |
| Viscosité Brookfield RVDVII+ (S 29 ; 5rpm) | | 159070c P | 150160 cP | 132720cP |

⇨ **Adapalène**

| Temps→ Conditions de stabilité↓ | | T0 | T+1mois | T+2mois |
|---|---|---|---|---|
| TA | g/g | 0.10 | 0.11 | 0.10 |
| | % titre attendu | 100 | 110 | 100 |
| 40°C | g/g | NA | 0.10 | 0.10 |
| | % titre attendu | NA | 100 | 100 |

☝ **Benzoyl peroxide**
| Temps→ Conditions de stabilité↓ | | T0 | T+1mois | T+2mois |
|---|---|---|---|---|
| TA | g/g | 2.7 | 2.8 | 2.7 |
| | % titre attendu | 108 | 112 | 108 |
| 40°C | g/g | NA | 2.6 | 2.6 |
| | % titre attendu | NA | 104 | 104 |

**Exemple 3 : Formulation de type gel crème fluide contenant de l'adapalène à 0.3% et du peroxyde de benzoyle à 1%**

La formule est préparée selon le mode opératoire décrit ci-dessus :

| Constituants | Teneur (% m/m) |
|---|---|
| Péroxyde de benzoyle | 1.00 |
| Adapalène | 0.30 |
| Lauroglycol | 2,00 |
| Synperonic PE/L62 | 0,20 |
| EDTA | 0.10 |
| Methylparaben | 0.20 |
| Methocel E4MPrenium | 0.10 |
| Carbopol ETD2020NF | 0.30 |
| Olépal isostéarique | 2.00 |
| Cosbiol | 8.00 |
| Cetiol SN PH | 8.00 |
| Propyl paraben | 0.05 |
| Sodium Hydroxyde 10% m/m | qsp pH5.5 +/- 0.5 |
| Eau purifiée | qsp 100% |

**Exemple 4 : Formulation de type gel crème fluide contenant de l'adapalène à 0.10% et du péroxyde de benzoyle à 0.25%**

La formule est préparée selon le mode opératoire décrit ci-dessus :

| Constituants | Teneur (% m/m) |
|---|---|
| Péroxyde de benzoyle | 0.25 |
| Adapalène | 0.10 |
| Propylène Glycol | 2,00 |

(suite)

| Constituants | Teneur (% m/m) |
| --- | --- |
| Synperonic PE/L62 | 0,20 |
| EDTA | 0.10 |
| Glycérine | 5.00 |
| Methylparaben | 0.20 |
| Carbopol Ultrez-20 | 0.30 |
| Veegum K | 0.20 |
| Gomme xanthane | 0.20 |
| ST-Cyclomethicone 5NF | 7.00 |
| Propyl paraben | 0.10 |
| Triethanolamine | qsp pH5.5 +/- 0.5 |
| Eau purifiée | qsp 100% |

**Revendications**

1. Composition comprenant, dans un milieu physiologiquement acceptable, de de l'adapalène et du péroxyde de benzoyle dispersé, **caractérisée en ce qu'**elle se présente sous forme de gel-crème et comprend les ingrédients (exprimé en pourcentage en poids) suivants:

    - de 0,001 à 5% d'adapalène ;
    - 0,025 à 10 %, de péroxyde de benzoyle ;
    - de 30% à 95% d'eau;
    - de 0,01% à 15% d'un ou plusieurs agents gélifiants pH-indépendants ;
    - de 5% à 30% de phase grasse comprenant au moins un composé lipophile choisi parmi les huiles végétales, minérales, animales, ou synthétiques, les huiles de silicones, et leurs mélanges;
    - de 0% à 1,5% d'agents chélatants ;
    - de 0% à 10% d'un ou plusieurs agents mouillants ;
    - de 0% à 3% d'agents conservateurs ;
    - de 0% à 20% d'agents humectants et/ou émollients ;
    - de 0% à 3% d'agents stabilisants ;
    - de 0% à 10% d'agents neutralisants.

2. Composition la revendication 1, **caractérisée en ce que** le péroxyde de benzoyle est sous forme encapsulée ou libre.

3. Composition selon l'une quelconque des revendications précédentes, **caractérisée en ce qu'**elle est dépourvue d'émulsionnant.

4. Composition selon l'une quelconque des revendications précédentes, **caractérisée en ce qu'**elle est stable physiquement et chimiquement.

5. Composition selon la revendication 1, **caractérisée en ce que** le composé lipophile est choisi parmi les huiles de paraffine, l'huile d'amande douce, l'huile de palme, l'huile de soja, l'huile de sésame, l'huile de tournesol, la lanoline, le squalène, l'huile de poisson, l'huile de vison, le squalane, le cétéaryl isononanoate, le diisopropyl adipate, le palmitate d'isopropyle, le diisopropyl adipate, le caprylique/caprique triglycéride, une huile de silicone volatile ou non volatile.

6. Composition selon l'une des revendications précédentes, **caractérisée en ce que** l'agent gélifiant est choisi parmi les composés de la famille des polyacrylamides ; les carbomers dits non sensibles aux électrolytes ; les polysaccharides; la cellulose et ses dérivés; et les silicates d'aluminium et de magnésium.

7. Composition selon la revendication 6, **caractérisée en ce que** l'agent gélifiant est choisi parmi le mélange Sodium acryloyldimethyltaurate copolymer / isohexadecane / polysorbate 80, le mélange polyacrylamide / isoparaffine C13-14 / laureth-7, les carbomers dits non sensibles aux électrolytes , la gomme de xanthane, l'hydroxypropylméthylcellulose et l'hydroxyéthylcellulose.

8. Composition selon l'une quelconque des revendications précédentes, **caractérisée en ce que** l'agent mouillant est choisi parmi un poloxamer et le propylène glycol.

9. Composition selon l'une quelconque des revendications précédentes, **caractérisée en ce qu'**elle comprend les ingrédients (exprimé en pourcentage en poids) suivants:

- de 0,01% à 0,5% d'adapalène ;
- de 2 à 10 %, de péroxyde de benzoyle ;
- de 50% à 85% d'eau;
- de 0,1% à 5% d'un ou plusieurs agents gélifiants pH-indépendants ;
- de 5% à 30% de phase grasse ;
- de 0,05% à 0.5% d'agents chélatants ;
- de 2% à 7 % d'un ou plusieurs agents mouillants ;
- de 0,05% à 1% d'agents conservateurs ;
- de 2% à 15% d'agents humectants et/ou émollients ;
- de 0,05% à 2% d'agents stabilisants ;
- de 0,1% à 5% d'agents neutralisants.

10. Composition selon l'une quelconque des revendications de 1 à 9 à titre de médicament.

11. Procédé de préparation de la composition selon l'une quelconque des revendications 1 à 9, **caractérisé en ce qu'**il comprend successivement les étapes suivantes :

a) mélange de l'adapalène avec de l'eau jusqu'à parfaite dispersion, afin d'obtenir la phase active 1;
b) mélange du péroxyde de benzoyle avec de l'eau jusqu'à parfaite dispersion, afin d'obtenir la phase active 2;
c) mélange d'au moins agent gélifiant pH-indépendant avec de l'eau, optionnellement un ou plusieurs agents chélatants, un ou plusieurs conservateurs, un ou plusieurs humectants et/ou émollients, un ou plusieurs agents stabilisants et les additifs hydrophiles afin d'obtenir la phase aqueuse ;
d) optionnellement, mélange d'au moins deux composés lipophiles afin d'obtenir la phase grasse ;
e) mélange des 2 phases actives obtenues en a) et b) pour obtenir une phase active unique ;
f) introduction de la phase active unique obtenue en e) dans la phase aqueuse obtenue en c) ;
g) introduction du composé unique de la phase grasse ou optionnellement de la phase grasse obtenue en d) afin d'obtenir un gel-crème ;
h) si nécessaire, les additifs thermosensibles sont ajoutés ;
i) si nécessaire, un agent de neutralisation du gélifiant est introduit dans le gel-crème obtenu en h);
j) si nécessaire un complément d'eau est ajouté.

12. Procédé de préparation de la composition selon la revendication 11, **caractérisé en ce qu'**il comprend successivement les étapes suivantes :

a) On mélange de l'adapalène avec au moins un agent mouillant, dans de l'eau, jusqu'à ce que de l'adapalène soit parfaitement dispersé, afin d'obtenir la phase active 1;
b) On mélange le péroxyde de benzoyle avec au moins un agent mouillant, dans de l'eau, jusqu'à ce qu'il soit parfaitement dispersé, afin d'obtenir la phase active 2;
c) On solubilise dans l'eau, sous agitation, si nécessaire à chaud, un ou plusieurs agent gélifiant pH-indépendant (à l'exception du polyacrylamide) et optionnellement, un ou plusieurs agents chélatants, un ou plusieurs conservateurs, un ou plusieurs humectants et/ou émollients, un ou plusieurs agents stabilisants et les additifs hydrophiles non thermosensibles. On maintient l'agitation et l'éventuel chauffage jusqu'à homogénéité pour obtenir la phase aqueuse ;
d) Optionnellement, on mélange si nécessaire à chaud au moins des huiles, et éventuellement des corps gras solides, et des conservateurs et les additifs lipophiles non thermosensibles jusqu'à homogénéité afin d'obtenir la phase grasse ;
e) On mélange les phases actives 1 et 2 de manière à obtenir une phase active unique ;
f) On additionne la phase active unique obtenue en e) à la phase aqueuse obtenue en c) ;
g) Optionnellement, on introduit le polyacrylamide à la phase obtenue en f) ;
h) L'unique constituant de phase grasse ou optionnellement, ladite phase grasse obtenue en d) est introduit dans la phase obtenue en f) ou g) afin d'obtenir un gel crème;
i)Si nécessaire, les additifs thermosensibles sont ajoutés ;

j) Si nécessaire, un agent de neutralisation du gélifiant est introduit dans le gel crème obtenu en f) afin d'obtenir le pH désiré,

k) Si nécessaire un complément d'eau est ajouté.

13. Utilisation d'une composition selon l'une quelconque des revendications 1 à 9 pour la fabrication d'une préparation pharmaceutique destinée à prévenir ou à traiter les affections dermatologiques liées à des désordres de la différentiation et/ou la prolifération cellulaire et/ou de la kératinisation.

14. Utilisation d'une composition selon l'une quelconque des revendications de 1 à 9 pour fabriquer une préparation pharmaceutique destinée à prévenir ou à traiter les acnés vulgaires.

15. Utilisation cosmétique non thérapeutique d'une composition selon l'une quelconque des revendications 1 à 9 pour faire repousser les cheveux ou éviter leur chute, pour lutter contre l'aspect gras de la peau ou des cheveux, ou pour prévenir et/ou lutter contre le vieillissement photo-induit ou chronologique.

16. Composition selon l'une quelconque des revendications 1 à 9 pour son utilisation pour le traitement des peaux à tendance acnéique ou pour la protection de la peau contre les aspects néfastes du soleil.

**Patentansprüche**

1. Zusammensetzung, die in einer physiologisch akzeptablen Umgebung Adapalen und dispergiertes Benzoylperoxid umfasst, **dadurch gekennzeichnet, dass** sie in Form von Cremegel ist und folgende Bestandteile (in Gewichtsprozent) umfasst:

   - 0,001 bis 5% Adapalen;
   - 0,025 bis 10% Benzoylperoxid;
   - 30% bis 95% Wasser;
   - 0,01% bis 15% ein oder mehrere pH-unabhängige Geliermittel;
   - 5% bis 30% Fettphase, die mindestens eine lipophile Verbindung umfasst, ausgewählt unter pflanzlichen, mineralischen, tierischen oder synthetischen Ölen, Silikonölen und deren Mischungen;
   - 0% bis 1,5% Chelatbildner;
   - 0% bis 10% ein oder mehrere Netzmittel;
   - 0% bis 3% Konservierungsstoffe;
   - 0% bis 20% Feuchthaltemittel und/oder Weichmacher;
   - 0% bis 3% Stabilisatoren;
   - 0% bis 10% Neutralisationsmittel.

2. Zusammensetzung nach Anspruch 1, **dadurch gekennzeichnet, dass** das Benzoylperoxid eingekapselt oder frei ist.

3. Zusammensetzung nach einem der vorherigen Ansprüche, **dadurch gekennzeichnet, dass** sie ohne Emulgator ist.

4. Zusammensetzung nach einem der vorherigen Ansprüche, **dadurch gekennzeichnet, dass** sie physikalisch und chemisch stabil ist.

5. Zusammensetzung nach Anspruch 1, **dadurch gekennzeichnet, dass** die lipophile Verbindung ausgewählt wird unter: Paraffinölen, mildes Mandelöl, Palmöl, Sojaöl, Sesamöl, Sonnenblumenöl, Lanolin, Squalen, Fischöl, Nerzöl, Squalan, Cetearylisononanoat, Diisopropyladipat, Isopropylpalmitat, Diisopropyladipat, Capric/Capryltriglycerid, ein flüchtiges oder nicht flüchtiges Silikonöl.

6. Zusammensetzung nach einem der vorherigen Ansprüche, **dadurch gekennzeichnet, dass** das Geliermittel unter den Verbindungen aus der Familie der Polyacrylamide; nicht elektrolytensensiblen Carbomere; Polysaccharide; Zellstoff und seine Derivate; und Aluminium- und Magnesiumsilikaten ausgewählt wird.

7. Zusammensetzung nach Anspruch 6, **dadurch gekennzeichnet, dass** das Geliermittel unter der Mischung Natriumacryloyldimethyltauratcopolymer / Isohexadecan / Polysorbat 80, der Mischung Polyacrylamide / Isoparaffin C13-14 / Laureth-7, den nicht elektrolytensensiblen Carbomeren, Xanthangummi, Hydroxypropylmethylcellulose und Hydroxyethylcellulose ausgewählt wird.

8. Zusammensetzung nach einem der vorherigen Ansprüche, **dadurch gekennzeichnet, dass** der Netzstoff unter einem Poloxamer und Propylenglykol ausgewählt wird.

9. Zusammensetzung nach einem der vorherigen Ansprüche, **dadurch gekennzeichnet, dass** sie folgende Bestandteile umfasst (ausgedrückt in Gewichtsprozent):

   - 0,01% bis 0,5% Adapalen;
   - 2 bis 10% Benzoylperoxid;
   - 50% bis 85% Wasser;
   - 0,1% bis 5% ein oder mehrere pH-unabhängige Geliermittel;
   - 5% bis 30% Fettphase;
   - 0,05% bis 0,5% Chelatbildner;
   - 2% bis 7 % ein oder mehrere Netzmittel;
   - 0,05% bis 1% Konservierungsstoffe;
   - 2% bis 15% Feuchthaltemittel und/oder Weichmacher;
   - 0,05% bis 2% Stabilisatoren;
   - 0,1% bis 5% Neutralisationsmittel.

10. Zusammensetzung nach einem der Patentansprüche 1 bis 9 als Medikament.

11. Verfahren zur Zubereitung der Zusammensetzung nach einem der Ansprüche 1 bis 9, **dadurch gekennzeichnet, dass** es nacheinander die folgenden Schritte umfasst:

   a) Mischen von Adapalen mit Wasser bis zur perfekten Dispersion, um die Aktivphase 1 zu erhalten;
   b) Mischen des Benzoylperoxids mit Wasser bis zur perfekten Dispersion, um die Aktivphase 2 zu erhalten;
   c) Mischen von mindestens einem pH-unabhängigen Geliermittel mit Wasser, optional einem oder mehreren Chelatbildnern, einem oder mehreren Konservierungsstoffen, einem oder mehreren Feuchthaltemitteln und/oder Weichmachern, einem oder mehreren Stabilisatoren und den hydrophilen Zusatzstoffen, um die wässerige Phase zu erhalten;
   d) optionales Mischen von mindestens zwei lipophilen Verbindungen, um die Fettphase zu erhalten;
   e) Mischen der 2 in a) und b) erhaltenen Aktivphasen, um eine einzige Aktivphase zu erhalten;
   f) Einführen der in e) erhaltenen einzigen Aktivphase in die in c) erhaltene wässerige Phase;
   g) Einführen der einzigen Verbindung der Fettphase oder optional der in d) erhaltenen Fettphase, um ein Cremegel zu erhalten;
   h) falls nötig werden die temperaturempfindlichen Zusatzstoffe hinzugefügt;
   i) falls nötig wird ein Wirkstoff zur Neutralisierung des Geliermittels in das in h) erhaltene Cremegel eingeführt;
   j) falls nötig wird eine Wasserergänzung hinzugefügt.

12. Verfahren zur Zubereitung der Zusammensetzung nach Anspruch 11, **dadurch gekennzeichnet, dass** es nacheinander die folgenden Schritte umfasst:

   a) Mischen von Adapalen mit mindestens einem Netzmittel, in Wasser, bis das Adapalen vollkommen dispergiert ist, um die Aktivphase 1 zu erhalten;
   b) Mischen des Benzoylperoxids mit mindestens einem Netzmittel, in Wasser, bis es vollkommen dispergiert ist, um die Aktivphase 2 zu erhalten;
   c) Auflösen in Wasser, unter Rühren, falls nötig erwärmt, eines oder mehrerer pH-unabhängigen Geliermittel (außer Polyacrylamiden) und optional eines oder mehrerer Chelatbildner, eines oder mehrerer Konservierungsstoffe, eines oder mehrerer Feuchthaltemittel und/oder Weichmacher, eines oder mehrerer Stabilisatoren und der nicht temperaturempfindlichen hydrophilen Zusatzstoffe. Das Rühren und das eventuelle Erwärmen werden bis zur Homogenität beibehalten, um die wässerige Phase zu erhalten;
   d) optionales Mischen, falls nötig erwärmt, mindestens von Ölen, und eventuell festen Fettkörpern, und Konservierungsstoffen und die nicht temperaturempfindlichen lipophile Zusatzstoffe bis zur Homogenität, um die Fettphase zu erhalten;
   e) Mischen der Aktivphasen 1 und 2, um eine einzige Aktivphase zu erhalten;
   f) Hinzufügen der einzigen in e) erhaltenen Aktivphase zu der in c) erhaltenen wässerigen Phase;
   g) optionales Einführen des Polyacrylamids in die in f) erhaltene Phase;
   h) Der einzige Bestandteil der Fettphase oder optional die besagte in d) erhaltene Fettphase wird in die in f) oder g) erhaltene Phase eingeführt, um ein Cremegel zu erhalten;

i) falls nötig werden die temperaturempfindlichen Zusatzstoffe hinzugefügt;
j) falls nötig wird ein Wirkstoff zur Neutralisierung des Geliermittels in das in f) erhaltene Cremegel eingeführt, um den gewünschten pH zu erhalten,
k) falls nötig wird eine Wasserergänzung hinzugefügt.

13. Verwendung einer Zusammensetzung nach einem der Ansprüche 1 bis 9 zum Herstellen eines pharmazeutischen Präparats, um Hauterkrankungen aufgrund von Störungen der Differenzierung und/oder Vermehrung der Zellen und/oder Keratinisierung vorzubeugen oder sie zu behandeln.

14. Verwendung einer Zusammensetzung nach einem der Patentansprüche 1 bis 9 zum Herstellen eines pharmazeutischen Präparats, um Akne vulgaris vorzubeugen oder sie zu behandeln.

15. Kosmetische, nicht therapeutische Verwendung einer Zusammensetzung nach einem der Ansprüche 1 bis 9, um das Haar wieder wachsen zu lassen oder seinen Ausfall zu vermeiden, um fettes Aussehen der Haut oder der Haare zu bekämpfen, oder um Lichtalterung oder chronologischer Alterung vorzubeugen und/oder sie zu bekämpfen.

16. Zusammensetzung nach einem der Ansprüche 1 bis 9 zur Verwendung für die Behandlung von zu Akne neigender Haut oder zum Schutz der Haut vor Schädigungen durch die Sonne.

**Claims**

1. Composition comprising, in a physiologically acceptable medium, adapalene and dispersed benzoyl peroxide, **characterized in that** it is in the form of a cream gel and comprises the following ingredients (expressed as a percentage by weight):

   - from 0.001 to 5% adapalene;
   - 0.025 to 10% benzoyl peroxide;
   - from 30% to 95% water;
   - from 0.01% to 15% of one or more pH-independent gelling agents;
   - from 5% to 30% fatty phase comprising at least one lipophilic compound chosen from vegetable, mineral, animal or synthetic oils, silicone oils, and their mixtures;
   - from 0% to 1.5% chelating agents;
   - from 0% to 10% of one or more wetting agents;
   - from 0% to 3% preservatives;
   - from 0% to 20% humectants and/or emollients;
   - from 0% to 3% stabilizing agents;
   - from 0% to 10% neutralizing agents.

2. Composition according to claim 1, **characterized in that** the benzoyl peroxide is in an encapsulated or free form.

3. Composition according to any of the preceding claims, **characterized in that** it does not contain any emulsifier.

4. Composition according to any of the preceding claims, **characterized in that** it is physically and chemically stable.

5. Composition according to claim 1, **characterized in that** the lipophilic compound is chosen from paraffin oils, sweet almond oil, palm oil, soybean oil, sesame oil, sunflower oil, lanolin, squalene, fish oil, mink oil, squalane, cetearyl isononanoate, diisopropyl adipate, isopropyl palmitate, diisopropyl adipate, caprylic/capric triglyceride, a volatile or non-volatile silicone oil.

6. Composition according to one of the preceding claims, **characterized in that** the gelling agent is chosen from compounds from the polyacrylamide family; so-called non-electrolyte sensitive carbomers; polysaccharides; cellulose and its derivatives; and aluminium and magnesium silicates.

7. Composition according to claim 6, **characterized in that** the gelling agent is chosen from the sodium acryloyldimethyltaurate copolymer/isohexadecane/polysorbate 80 mixture, the polyacrylamide/isoparaffin C13-14/laureth-7 mixture, so-called non-electrolyte sensitive carbomers, xanthan gum, hydroxypropylmethylcellulose and hydroxyethylcellulose.

8. Composition according to any of the preceding claims, **characterized in that** the wetting agent is chosen from a poloxamer and propylene glycol.

9. Composition according to any of the preceding claims, **characterized in that** it comprises the following ingredients (expressed as a percentage by weight):

    - from 0.01% to 0.5% adapalene;
    - from 2 to 10% benzoyl peroxide;
    - from 50% to 85% water;
    - from 0.1% to 5% of one or more pH-independent gelling agents;
    - from 5% to 30% fatty phase;
    - from 0.05% to 0.5% chelating agents;
    - from 2% to 7% of one or more wetting agents;
    - from 0.05% to 1% preservatives;
    - from 2% to 15% humectants and/or emollients;
    - from 0.05% to 2% stabilizing agents;
    - from 0.1% to 5% neutralizing agents.

10. Composition according to any of claims 1 to 9 for use as a medicament.

11. Method for preparing the composition according to any of claims 1 to 9, **characterized in that** it successively comprises the following steps:

    a) mixing the adapalene with water until perfect dispersion, in order to obtain the active phase 1;
    b) mixing the benzoyl peroxide with water until perfect dispersion, in order to obtain the active phase 2;
    c) mixing at least the pH-independent gelling agent with water, optionally one or more chelating agents, one or more preservatives, one or more humectants and/or emollients, one or more stabilizing agents and the hydrophilic additives in order to obtain the aqueous phase;
    d) optionally, mixing at least two lipophilic compounds in order to obtain the fatty phase;
    e) mixing the 2 active phases obtained in a) and b) in order to obtain a single active phase;
    f) adding the single active phase obtained in e) to the aqueous phase obtained in c);
    g) adding the single compound from the fatty phase or optionally the fatty phase obtained in d) in order to obtain a cream gel;
    h) if necessary, thermosensitive additives are added;
    i) if necessary, a gelling agent neutralizing agent is added to the cream gel obtained in h)
    j) if necessary, additional water is added.

12. Method for preparing the composition according to claim 11, **characterized in that** it successively comprises the following steps:

    a) The adapalene is mixed with at least one wetting agent, in water, until the adapalene is perfectly dispersed, in order to obtain the active phase 1;
    b) The benzoyl peroxide is mixed with at least one wetting agent, in water, until it is perfectly dispersed, in order to obtain the active phase 2;
    c) One or more pH-independent gelling agents (with the exception of polyacrylamide) and optionally one or more chelating agents, one or more preservatives, one or more humectants and/or emollients, one or more stabilizing agents and the non-thermosensitive hydrophilic additives are solubilized in water, under stirring, under heating if necessary. Stirring and eventual heating are maintained until homogeneity is reached in order to obtain the aqueous phase;
    d) Optionally, at least oils, and possibly solid fatty substances, and preservatives and non-thermosensitive lipophilic additives, are mixed, if necessary under heating, until homogeneity is reached in order to obtain the fatty phase;
    e) The active phases 1 and 2 are mixed so as to obtain a single active phase;
    f) The single active phase obtained in e) is added to the aqueous phase obtained in c);
    g) Optionally, the polyacrylamide is added to the phase obtained in f);
    h) The single constituent of the fatty phase or optionally said fatty phase obtained in d) is added to the phase obtained in f) or g) in order to obtain a cream gel;
    i) If necessary, thermosensitive additives are added;

j) If necessary, a gelling agent neutralizing agent is added to the cream gel obtained in f) in order to obtain the desired pH,

k) If necessary, additional water is added.

13. Use of a composition according to any of claims 1 to 9 in the production of a pharmaceutical preparation intended for preventing or treating dermatological conditions associated with cell differentiation and/or proliferation and/or keratinization disorders.

14. Use of a composition according to any of claims 1 to 9 in the production of a pharmaceutical preparation intended for preventing or treating acne vulgaris.

15. Non-therapeutic cosmetic use of a composition according to any of claims 1 to 9 for promoting hair regrowth or preventing hair loss, combating the oily appearance of the skin or hair, or preventing and/or fighting against photo-induced or chronological aging.

16. Composition according to any of claims 1 to 9 for using in the treatment of acneprone skin or for the protection of the skin against the harmful aspects of the sun.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 03055472 A

**Littérature non-brevet citée dans la description**

- **CUNLIFFE W.J.** *J. Dermatol. Treat.,* 2000, vol. 11, 13-14
- **CHELLQUIST E.M. ; GORMAN W.G.** *Pharm. Res.,* 1992, vol. 9, 1341-1346
- **B. MARTIN et al.** *Br.J.Dermatol.,* 1998, vol. 139 (52), 8-11
- **B.MARTIN et al.** *Br.J.Dermatol.,* 1998, vol. 139 (52), 8-11
- **BOLLINGER.** *Journal of Pharmaceutical Science,* 1977, vol. 5